(19) Europäisches Patentamt
European Patent Office
Office européen des brevets

(11) **EP 3 398 971 A1**

(12) **EUROPEAN PATENT APPLICATION**
published in accordance with Art. 153(4) EPC


(43) Date of publication:
**07.11.2018 Bulletin 2018/45**

(21) Application number: **15911826.4**

(22) Date of filing: **30.12.2015**

(51) Int Cl.:
***C08B 37/10*** *(2006.01)* ***A61K 31/727*** *(2006.01)*
***A61P 35/00*** *(2006.01)*

(86) International application number:
**PCT/CN2015/099901**

(87) International publication number:
**WO 2017/113197 (06.07.2017 Gazette 2017/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**



(71) Applicant: **Shenzhen Hepalink Pharmaceutical Group Co., Ltd.**
**Shenzhen, Guangdong 518057 (CN)**

(72) Inventor: **LI, Li**
**Shenzhen**
**Guangdong 518057 (CN)**

(74) Representative: **Hoffmann Eitle Patent- und Rechtsanwälte PartmbB**
**Arabellastraße 30**
**81925 München (DE)**


(54) **SULFATED HEPARIN OLIGOSACCHARIDE AND PREPARATION METHOD AND APPLICATION THEREOF**


(57) Provided are a sulfated heparin oligosaccharide as well as a preparation method and an application thereof. The sulfated heparin oligosaccharide is characterized in that a non-reducing end of a molecule of the sulfated heparin oligosaccharide contains an unsaturated double bond resulting from enzymolysis by heparinase, as well as a uronic acid derivative and a glycosylamine derivative; the sulfated heparin oligosaccharide has a structure represented by formula I, wherein $R_1$, $R_2$, $R_3$, $R_4$, $R_5$, $R_6$, $R_7$, $R_8$, $R_9$, $R_a$, $R_b$, $R_c$ and $R_d$ are independently $SO_3^-$ or H; $R_x$', $R_y$' and $R_z$' are independently $COCH_3$ or $SO_3^-$, and n is 1-3. The preparation method obtains a sulfated oligosaccharide with a controllable degree of sulfation. The sulfated heparin oligosaccharide has a high activity for inhibiting heparanase in vitro, with an activity 4-5 times higher than that of heparin for inhibiting cell adhesion and migration, and an activity 2-3 times higher than that of heparin for resisting tumor metastasis in mice, thus having a relatively good effect in resisting tumor metastasis and a relatively high specificity.


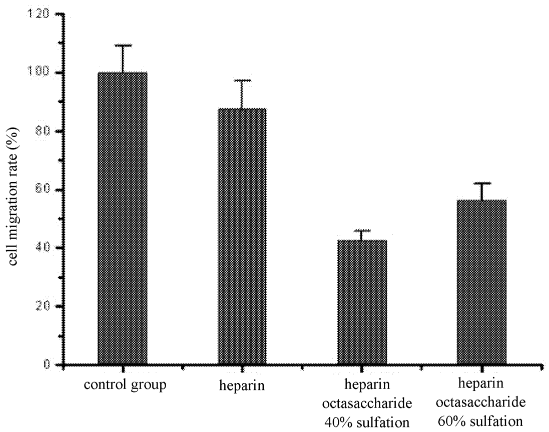


FIG.8

EP 3 398 971 A1

**Description**

**FIELD OF THE INVENTION**

The present invention is in the field of anti-tumor agents, and relates to a sulfated heparin oligosaccharide and a preparation method and use thereof.

**BACKGROUND OF THE INVENTION**

Tumors are a major threat to human health, more seriously, malignant tumors, such as liver cancer and lung cancer *etc.* are prone to metastasis. Currently, there are no drugs for inhibiting tumor metastasis, making treatment for tumor metastasis difficult, and thus making tumor metastasis the most significant cause of death in cancer patients. Two processes are very critical for achieving invasion and migration of tumor cells: one is to break through a barrier formed by the extracellular matrix (ECM) and the basement membrane (BM), and the other is to form new blood vessels. ECM and BM are barriers against invasion and metastasis of tumor cells, and malignant tumor cells must enter into the circulation across the ECM and BM, so as to spread and metastasize. In this process, the degradation of various components of the ECM and heparin sulfate proteoglycans (HSPGs) is essential. Heparanase (HPA) is the only endo-β-D-glucuronidase currently found in mammals that can cleave heparan sulfate (HS). HPA identifies the specific structure of HS, and it does not completely cleaves the HS side chain, but only cleaves the glycosidic bonds at certain sites in the HS side chain, which is degraded as short saccharide chains of 10 to 15 saccharide units. In addition, HS side chains can bind a variety of biologically active molecules, such as growth factors, cytokines, chemokines, morphogens and blood coagulating proteins. HPA degrades HS to release active growth factors which can promote tumor angiogenesis, and tumor growth, invasion and metastasis. Therefore, HPA plays an important role in the invasion and metastasis of tumor cells, and the research and screening of HPA inhibitors have become a new direction for humans to seek potential drugs for the treatment of cancers.

For a long time, clinical evidence shows that heparin has an anti-tumor effect, and in recent years, clinical trials for low molecular weight heparin-assisted anti-tumor therapy are undergoing. After long-term research, the anti-tumor effect of heparin has been widely recognized and appreciated in the industry, and it might be realized mainly by inhibiting tumor metastasis, and further be realized by inhibiting the heparanase activity *in vivo.*

Heparin, as a traditional anticoagulant, is mainly used for its anticoagulant activity. However, it has a broad range of biological activity due to the structural diversity thereof. In non-anticoagulant applications of heparin, its anticoagulant activity is a common and important side effect. As such, the anticoagulant activity of heparin is a major disadvantageous factor in the application of anti-tumor metastasis, and it is prone to cause side effects such as bleeding. An important aspect in the research of the application of the non-anticoagulant activity of heparin is to destroy the anticoagulant activity while maintaining the basic structure of heparin. With respect to anti-tumor metastasis, heparin has high inhibitory activity on heparanase, but has no significant effect on the inhibition of tumor growth and on the inhibitory activity on tumor cell invasion and adhesion at the cellular level, and achieves poor results in an anti-tumor metastasis experiment in mice and thus cannot significantly inhibit the occurrence of tumor metastasis. The main reason therefor might be that the specificity of heparin is not high, and thus heparin can interact with a variety of endogenous substances, reducing its ability to bind to heparanase.

CN101824100A discloses a heparin oligosaccharide dodecamer having the following structural formula:

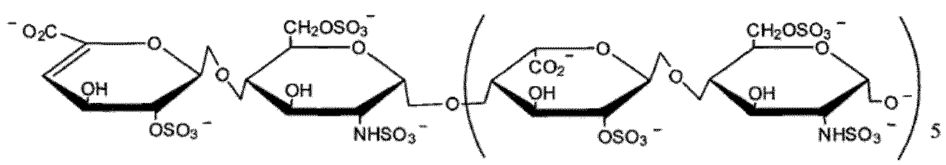

,

which has the use of preventing proliferation of vascular smooth muscle cells. However, the oligosaccharide of that invention has no significant ability of inhibiting tumor cell adhesion and migration.

CN 104764847A discloses a method of preparing a heparin oligosaccharide comprising a N-acetylated structure, and the following 4 hexasaccharide fragments and 3 octasaccharide fragments are disclosed in this reference:

dp6a Δ HexA-GlcNS-HexA-GlcNS-HexA2S-GlcNS6S :
dp6b Δ HexA2S-GlcNS6S-HexA2S-GlcNAc-HexA2S-GlcNS6S :
dp6c Δ HexA-GlcNS6S-HexA-GlcNAc6S-HexA2S-GlcNS6S :

dp6d Δ Hexk-GlcNS6S-HexA2S-GlcNAc6S-HexA2S-GlcNS6S:
dp8a Δ HexA2S-GlcNS-[HexA2S-GlcNAc-HexA-GleNS]-HexA2S-GlcNS6S :
dp8b Δ HexA2S-GlcNS6S-[HexA2S-GlcNAc-HexA-GlcNS]-HexA2S-GlcNS6S :
dp8c Δ HexA2S-GlcNS6S-[HexA 2S-GlcNAc-HexA-GlcNAc6S]-HexA2S-Glc-NS6S:

That invention merely address the difficulties in the preparation and structural verification of a heparin oligosaccharide comprising a N-acetylated structure, and achieves no significant improvement in the heparanase inhibitory activity *in vivo.*

As such, in the art, it is desired to obtain a heparin oligosaccharide having heparanase inhibitory activity *in vivo* and reduced anticoagulant activity.

## SUMMARY OF THE INVENTION

To address the disadvantages of the prior art, the object of the present invention is to provide a sulfated heparin oligosaccharide and a preparation method and use thereof.

To accomplish the object of the present invention, the following technical solution is employed in the present invention.

In the first aspect, the present invention provides a sulfated heparin oligosaccharide, containing an unsaturated double bond resulting from enzymolysis by heparinase at the non-reducing end thereof, comprising an uronic acid derivative and a glycosylamine derivative, and having a structure represented by Formula I:

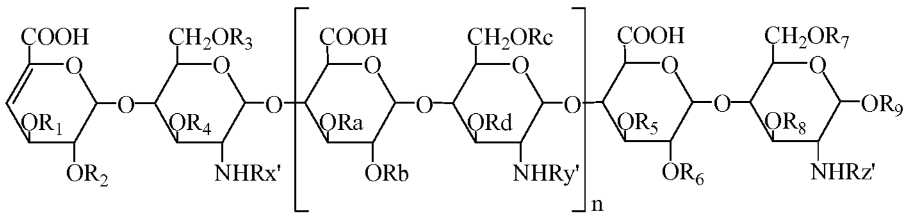

Formula I

wherein $R_1$, $R_2$, $R_3$, $R_4$, $R_5$, $R_6$, $R_7$, $R_8$, $R_9$, $R_a$, $R_b$, $R_c$ and $R_d$ are independently $SO_3^-$ or H; $R_x$', $R_y$' and $R_z$' are independently $COCH_3$ or $SO_3^-$, and n is 1 to 3.

In the present invention, n is 1 to 3, i.e., 1, 2, or 3. When n=1, the sulfated heparin oligosaccharide is sulfated heparin hexasaccharide; when n=2, the sulfated heparin oligosaccharide is sulfated heparin octasaccharide; and when n=3, the sulfated heparin oligosaccharide is sulfated heparin decasaccharide.

There is a double bond in the structure of the sulfated heparin oligosaccharide of the present invention, which thus has a strong absorption peak in the ultraviolet region at about 232 nm, which can be very conveniently taken advantage of in the qualitative and quantitative determination of the heparin oligosaccharide.

The sulfated heparin oligosaccharide of the present invention has good inhibitory activity on heparanase and tumor metastasis. It has a short saccharide chain and low molecular weight, and has no anticoagulant activity at all. Meanwhile, the sulfated heparin oligosaccharide has high specificity, and thus can specifically inhibit heparanase and inhibit tumor metastasis.

In the sulfated heparin oligosaccharide of the present invention, in Formula I, the number of sulfonic groups in each disaccharide unit is no less than 2 on average, for example, the number of sulfonic groups in each disaccharide unit can be 2, 3, 4, or 5 on average; the number of acetyl groups in each disaccharide unit is no more than 0.5 on average, for example, it can be 0.5 or 0.4; and the numbers of sulfonic groups at position 6 and position 3 in glucosamine in each disaccharide unit are both no less than 0.5 on average, for example, it can be 0.5, 0.8, 1, 1.5, or 2.

The number of groups (for example, sulfonic groups, acetyl groups, or sulfonic groups at position 6 and position 3 in glucosamine) in each disaccharide unit refers to the number that is obtained by averaging the number of sulfonic groups in the whole heparin oligosaccharide to each disaccharide unit, for example, if there are a total of 2 acetyl groups in a sulfated heparin octasaccharide, the number of acetyl groups in each disaccharide unit is 0.5 on average.

Preferably, the uronic acid in Formula I is glucuronic acid or iduronic acid.

Preferably, the cation forming a salt with the carboxyl and/or sulfonic group contained in Formula I is selected from the group consisting of $Na^+$, $K^+$ or $Ca^{2+}$.

In the above structural formula, the carboxyl or sulfonic group in glucuronic acid is negatively charged, and thus generally forms a salt with certain cations, typically $Na^+$, $K^+$ and $Ca^{2+}$.

Preferably, the sulfated heparin oligosaccharide is any one of, or a combination of at least two of, the compounds having the following structures:

DP6, a heparin hexasaccharide derivative:

$\Delta U_{3S}$-$A_{NS3S}$-I-$A_{NS3S}$-$G_{3S}$-$A_{NS3S6S}$

$\Delta U_{3S}$-$A_{NS}$-$I_{3S}$-$A_{NS3S}$-$G_{3S}$-$A_{NS3S6S}$

$\Delta U_{2S3S}$-$A_{NS3S6S}$-$I_{3S}$-$A_{NS3S}$-$G_{2S3S}$-$A_{NS3S6S}$

$\Delta U_{2S3S}$-$A_{NS3S6S}$-$I_{3S}$-$A_{NS3S6S}$-$G_{3S}$-$A_{NS3S6S}$

$\Delta U_{2S3S}$-$A_{NS3S6S}$-$I_{3S}$-$A_{NS3S6S}$-$G_{2S3S}$-$A_{NS3S6S}$

$\Delta U_{2S3S}$-$A_{NS3S6S}$-$I_{2S3S}$-$A_{NS3S6S}$-$G_{2S3S}$-$A_{NS3S6S}$

DP8, a heparin octasaccharide derivative:

$\Delta U_{3S}$-$A_{NS3S}$-I-$A_{NS3S}$-$I_{3s}$-$A_{NS6S}$-$G_{3S}$-$A_{NS3S6S}$

$\Delta U_{3S}$-$A_{NS}$-$I_{3S}$-$A_{NS3S}$-$I_{3S}$-$A_{NS6S}$-$G_{3S}$-$A_{NS3S6S}$

$\Delta U_{2S3S}$-$A_{NS3S6S}$-$I_{3S}$-$A_{NS3S}$-$I_{3S}$-$A_{NS6S}$-$G_{2S3S}$-$A_{NS3S6S}$

$\Delta U_{2S3S}$-$A_{NS3S6S}$-$I_{3S}$-$A_{NS3S6S}$-$I_{3S}$-$A_{NS6S}$-$G_{3S}$-$A_{NS3S6S}$

$\Delta U_{2S3S}$-$A_{NS3S6S}$-$I_{3S}$-$A_{NS3S6S}$-$I_{3S}$-$A_{NS6S}$-$G_{2S3S}$-$A_{NS3S6S}$

$\Delta U_{2S3S}$-$A_{NS3S6S}$-$I_{2S3S}$-$A_{NS3S6S}$-$I_{2S3S}$-$A_{NS6S}$-$G_{2S3S}$-$A_{NS3S6S}$

DP10, a heparin decasaccharide derivative:

$\Delta U_{3S}$-$A_{NS3S}$-I-$A_{NS3S}$-I-$A_{NS6S}$-I-$A_{NS6S}$-$G_{3S}$-$A_{NS3S6S}$

$\Delta U_{3S}$-$A_{NS}$-$I_{3S}$-$A_{NS3S}$-$I_{3S}$-$A_{NS6S}$-$I_{3S}$-$A_{NS6S}$-$G_{3S}$-$A_{NS3S6S}$

$\Delta U_{2S3S}$-$A_{NS3S6S}$-$I_{3S}$-$A_{NS3S}$-$I_{3S}$-$A_{NS6S}$-$I_{3S}$-$A_{NS6S}$-$G_{3S}$-$A_{NS3S6S}$

$\Delta U_{2S3S}$-$A_{NS3S6S}$-$I_{3S}$-$A_{NS3S6S}$-$I_{3S}$-$A_{NS6S}$-$I_{3S}$-$A_{NS6S}$-$G_{2S3S}$-$A_{NS3S6S}$

$\Delta U_{2S3S}$-$A_{NS3S6S}$-$I_{2S3S}$-$A_{NS3S6S}$-$I_{2S3S}$-$A_{NS6S}$-$I_{2S3S}$-$A_{NS6S}$-$G_{2S3S}$-$A_{NS3S6S}$

$\Delta U_{2S3S}$-$A_{NS3S6S}$-$I_{2S3S}$-$A_{NS3S6S}$-$I_{2S3S}$-$A_{NS3S6S}$-$I_{2S3S}$-$A_{NS3S6S}$-$G_{2S3S}$-$A_{NS3S6S}$

In the above structures, $\Delta$U represents

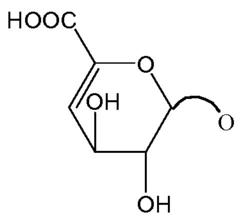

,

**I** represents $\alpha$-L-iduronic acid, **G** represents $\beta$-D-glucuronic acid, and **A** represents $\alpha$-D-glucosamine. NS represents a sulfonic group on the amino group, and 2S, 3S, 6S, *etc.* represent sulfonic groups at the 2-O, 3-O, and 6-O positions on the saccharide ring.

In the second aspect, the present invention provides a method of preparing the sulfated heparin oligosaccharide as described in the first aspect, comprising the following steps:

(1) degrading heparin with heparinase, separating and purifying to obtain a heparin oligosaccharide; and

(2) sulfating the heparin oligosaccharide obtained in step (1) with a sulfating reagent to obtain the sulfated heparin oligosaccharide.

In the method of preparing the sulfated heparin oligosaccharide of the present invention, the heparinase in step (1) is heparinase I.

In the method of preparing the sulfated heparin oligosaccharide of the present invention, a buffer, preferably a Tris-HCl buffer, pH 7.0, needs to be added when heparin is degraded with heparinase in step (1).

Preferably, the amount of heparinase added in step (1) is 15 to 25 IU/g heparin, e.g., 16 IU/g heparin, 16.5 IU/g heparin, 17 IU/g heparin, 17.5 IU/g heparin, 18 IU/g heparin, 18.5 IU/g heparin, 19 IU/g heparin, 19.5 IU/g heparin, 20 IU/g heparin, 21.5 IU/g heparin, 22 IU/g heparin, 23 IU/g heparin or 24 IU/g heparin, and preferably 18 to 23 IU/g heparin.

Preferably, the temperature for the degradation of heparin with heparinase in step (1) is 4°C to 37°C, e.g., 5°C, 8°C, 10°C, 12°C, 15°C, 18°C, 20°C, 22°C, 25°C, 28°C, 30°C, 32°C, 35°C or 37°C, and preferably 8°C to 25°C.

Preferably, the time for the degradation in step (1) is 8 to 24 hours, e.g., 9 hours, 10 hours, 11 hours, 12 hours, 13 hours, 14 hours, 15 hours, 16 hours, 17 hours, 18 hours, 19 hours, 20 hours, 21 hours, 22 hours or 23 hours, and preferably 10 to 20 hours.

Preferably, the degradation of heparin with heparinase in step (1) comprises inactivation at 95°C for 5 to 10 min, e.g., 5.5 min, 6 min, 6.5 min, 7 min, 7.5 min, 8 min, 8.5 min, 9 min, 9.5 min or 9.8 min, preferably 5 to 8 min, and more preferably 6 min, after the degradation.

Preferably, the separation in step (1) comprises ultrafiltration, preferably ultrafiltration with a 10 KDa ultrafiltration centrifuge tube.

Preferably, the purification in step (1) is separation and purification through column chromatography.

After separation through column chromatography, heparin oligosaccharide is obtained through processes such as concentration, desalting and lyophilization.

In the method of preparing the sulfated heparin oligosaccharide of the present invention, before the sulfation in step (2), the heparin oligosaccharide obtained in step (1) is subjected to swelling treatment.

Preferably, the solvent used for the swelling treatment is DMF.

Preferably, the sulfating reagent in step (2) is $(CH_3)_3N{\bullet}SO_3$.

Preferably, relative to 1 g of the heparin oligosaccharide, the amount of the sulfating reagent is 1 to 10 g, e.g., 1.2 g, 1.5 g, 2 g, 2.5 g, 3 g, 3.5 g, 4 g, 4.5 g, 5 g, 5.5 g, 6 g, 6.5 g, 7 g, 7.5 g, 8 g, 8.5 g, 9 g, 9.5 g or 9.8 g.

Preferably, the temperature for the sulfation in step (2) is 60°C to 120°C, e.g., 63°C, 65°C, 70°C, 73°C, 75°C, 78°C, 80°C, 83°C, 85°C, 88°C, 90°C, 93°C, 95°C, 98°C, 100°C, 115°C, 118°C or 120°C.

Preferably, the time for the sulfation in step (2) is 1 to 12 hours, e.g., 1.5 hours, 2 hours, 2.3 hours, 2.5 hours, 2.8 hours, 3 hours, 3.5 hours, 4 hours, 4.5 hours, 5 hours, 6 hours, 7 hours, 8 hours, 9 hours, 10 hours, 10.5 hours, 11 hours or 11.5 hours.

In the present invention, the sulfation degree of the heparin oligosaccharide can be controlled by controlling the ratio of the heparin oligosaccharide to the sulfating reagent, the reaction temperature, and the reaction time, so as to obtain heparin oligosaccharides with various degrees of sulfate group substitution.

In the present invention, after the sulfation of the heparin oligosaccharide obtained in step (1) with the sulfating reagent, the reaction mixture needs to be worked up and purified, that is, the reaction mixture is added with 10-fold volume of purified water to dissolve the precipitate, transferred to a dialysis bag with a molecular weight cut-off of 100 to 500 Da, desalted with a P10 column after dialysis for three days, concentrated before cationic impurities are removed with a cation exchange column, neutralized with high purity NaOH, KOH or $Ca(OH)_2$, *etc.,* and concentrated before lyophilization or precipitation with ethanol, so as to obtain the corresponding sulfated heparin oligosaccharide.

As a preferred technical solution of the present invention, the method of preparing the sulfated heparin oligosaccharide of the present invention comprises the following steps:

(1) degrading heparin with heparinase I added at an amount of 15 to 25 IU/g heparin at 4°C to 37°C for 8 h to 24 h, in a buffer being a Tris-HCl buffer, pH 7.0, and after the degradation, inactivating at 95°C for 5 to 10 min, ultrafiltering with a 10 KDa ultrafiltration centrifuge tube, and then separating and purifying through column chromatography to obtain the heparin oligosaccharide; and

(2) sulfating the heparin oligosaccharide obtained in step (1) at 60°C to 120°C for 1 to 12 hours with a sulfating reagent to obtain the sulfated heparin oligosaccharide.

The preparation method of the present invention can be employed to obtain sulfated oligosaccharides with various sulfation degrees. Each saccharide chain of the sulfated heparin oligosaccharide contains a double bond resulting from an elimination reaction of enzymolysis by heparinase at the non-reducing end thereof. Due to the presence of a double bond in the structure, the oligosaccharide has a strong absorption peak in the ultraviolet region at about 232 nm, which can be very conveniently taken advantage of in the qualitative and quantitative determination of the heparin oligosaccharide. The double bond does not substantially change during the sulfation of the heparin oligosaccharide. Heparin *per se* has no strong characteristic absorption peaks, and thus heparin substances can be detected only with DMB staining *etc.,* which, however, has low sensitivity and cannot be accommodated to complex systems. As such, the presence of the double bond greatly facilitates the detection of the heparin oligosaccharide and derivatives thereof, and can also play an important role in the detection of biological metabolic processes.

In a third aspect, the present invention provides use of the sulfated heparin oligosaccharide as described in the first aspect in the manufacture of a medicament for anti-tumor metastasis.

The sulfated heparin oligosaccharide of the present invention has good inhibitory activity on heparanase and tumor metastasis, and can be used as an anti-tumor drug or as an anti-tumor active ingredient for the manufacture of an anti-tumor medicament for the treatment of a tumor, and for the prevention and inhibition of tumor metastasis.

In the sulfated heparin oligosaccharide of the present invention, heparin hexasaccharide, heparin octasaccharide and heparin decasaccharide with various sulfonation degrees all have significant inhibitory activity. At a same sulfonation degree, different chain lengths will lead to different inhibitory activity on heparanase, the inhibitory activity levels are specifically: sulfonated heparin hexasaccharide < sulfonated heparin decasaccharide < sulfonated heparin octasaccharide. At a same chain length, different sulfation degrees of the heparin oligosaccharides lead to different inhibitory activity on heparanase, specifically: low sulfated (10 to 40%) heparin oligosaccharide < highly sulfated (60 to 90%) heparin oligosaccharide < moderately sulfated (40 to 60%) heparin oligosaccharide. In the structure of the sulfated heparin oligosaccharide, sulfonation at position 6 in glycosylamine and sulfonation at position 3 in glycosylamine contribute more to the heparanase inhibitory activity.

Compared with the prior art, the present invention achieves the following advantageous effects.

The sulfated heparin oligosaccharide of the present invention has good inhibitory activity on heparanase and tumor metastasis, comprises short saccharide chains and a low molecular weight, and has no anticoagulant activity at all with no anticoagulant activity and bleeding risks. The preparation method of the present invention can prepare a sulfated oligosaccharide with a controllable sulfation degree, which has high inhibitory activity on heparanase *in vitro,* inhibitory activity on cell adhesion and migration that is 4 to 5 times higher than that of heparin, and *in vivo* activity of anti-tumor metastasis in mice that is 2 to 3 times higher than that of the heparin, thereby having a good anti-tumor metastasis effect and high specificity.

## BRIEF DESCRIPTION OF THE DRAWINGS

FIG. 1 is a graph showing the results of separation of the heparin oligosaccharides by a Bio-Gel P-10 (2.5×100 cm) chromatographic column in Example 1 of the present invention;

FIG. 2 is a graph showing the molecular weight distribution of the heparin oligosaccharides with a same degree of polymerization in Example 1 of the present invention;

FIG. 3 is a total ion spectrum in the UPLC-MS of the heparin decasaccharide obtained in Example 1 of the present invention;

FIG. 4 is a total ion spectrum (partial) in the UPLC-MS of the heparin decasaccharide obtained in Example 1 of the present invention, and the attribution of corresponding peaks;

FIG. 5 shows HSQC spectra and the corresponding one-dimensional hydrogen spectra of the heparin octasaccharide (A) and 40% sulfated heparin octasaccharide (B) (δH ppm 6.1 to 3.165/δC ppm 112 to 52.6);

FIG. 6 is a graph showing the results of the inhibitory activity of the sulfated heparin oligosaccharides on heparanase;

FIG. 7 is a graph showing the results of the inhibitory activity of the sulfated heparin oligosaccharide on cell adhesion of HeLa cells;

FIG. 8 is a graph showing the results of the inhibitory activity of the sulfated heparin oligosaccharide on cell migration

of HeLa cells.

## DETAILED DESCRIPTION OF THE INVENTION

The technical solutions of the present invention will now be further illustrated by the following specific embodiments. As can be appreciated by a person skilled in the art, the Examples are given merely to aid understanding of the invention, and should not be considered limitative on the invention.

Example 1. Preparation of heparin oligosaccharides

24 g of heparin was taken and added with 240 mL of a Tris-HCl buffer solution, which was stirred to dissolve, added with 480 IU of heparinase I, stirred until homogeneous, and underwent an enzymatic reaction at 10°C for 16 h. After the reaction was complete, the reaction mixture was heated to 95°C for inactivation for 6 min, and ultrafiltered with a 10 KDa ultrafiltration centrifuge tube. The filtrate was separated with a Bio-Gel P-10 (2.5×100 cm) chromatographic column with 0.2 M $NH_4HCO_3$ as the eluent, 1.75 to 2.15-fold column volume of fractions was collected to obtain a heparin tetrasaccharide mixture, 1.35 to 1.75-fold column volume of fractions was collected to obtain a heparin hexasaccharide mixture, 1.05 to 1.35-fold column volume of fractions was collected to obtain a heparin octasaccharide mixture, 0.85 to 1.05-fold column volume of fractions was collected to obtain a heparin decasaccharide mixture, and 0.75 to 0.85-fold column volume of fractions was collected to obtain a heparin dodecasaccharide mixture. After removal of $NH_4HCO_3$ through rotary evaporation, the corresponding heparin oligosaccharides were obtained by lyophilization.

As shown in FIG. 1 which is a graph showing the separation of the heparin oligosaccharides by a Bio-Gel P-10 (2.5 x 100 cm) chromatographic column, it can be seen that after separation by the P10 column, heparin oligosaccharides are separated into different peaks based on different molecular weights, and oligosaccharides with different degrees of polymerization can be obtained by collecting fractions at the tips of the peaks.

FIG. 2 shows the molecular weight distribution of the obtained heparin oligosaccharides with a same degree of polymerization. It can be seen from the graph that the molecular weight distribution peaks of the oligosaccharides are sharp and symmetrical, indicating that the molecular weight distribution of the oligosaccharides was in narrow ranges, and meanwhile the molecular weight distribution gradually decreases from dodecasaccharide to tetrasaccharide, indicating that the oligosaccharides met the quality requirements.

The structures of the heparin oligosaccharides were characterized and verified by means of UPLC-MS *etc.* For example, FIG. 3 is a total ion mass spectrum in the UPLC-MS of the heparin decasaccharide obtained in the present invention, and FIG. 4 is a graph showing the attribution of corresponding peaks in the total ion spectrum of the heparin decasaccharide, wherein in ΔUx,y,z (specific values of x, y and z are given in the graph), x represents the number of saccharide units in an oligosaccharide chain, y represents the total number of sulfonic groups in an oligosaccharide chain, z represents the total number of acetyl groups in an oligosaccharide chain; and LR represents the linking region. According to the structure attribution in Fig. 4, in the heparin decasaccharides, the oligosaccharides are mostly heparin decasaccharides, with only a small portion being highly sulfated heparin octasaccharides. The above characterization results indicate that the preparation of oligosaccharides is successful and meets the purity requirements.

Example 2. Preparation of 20% to 80% sulfated heparin octasaccharides

0.54 g of the starting material of heparin octasaccharide was weighed and transferred to a reaction flask, added with 25 mL of anhydrous DMF, and stirred to dissolve. With stirring, 0.86 g of $(CH_3)_3N{\bullet}SO_3$ was weighed and gradually added to the above solution, and stirred for 10 min. The reaction flask was capped appropriately, placed in an oil bath at 80°Cn and stirred for 4 h. The reaction was stopped before left to cool to room temperature. The solid was dissolved in 90 mL of purified water, and the pH was adjusted to near neutral with 2 M NaOH. The solution was transfer to a dialysis bag with a molecular weight cut-off of 100 to 500 Da, and after three days of dialysis, the dialysate was detected with $BaCl_2$ to determine whether a large amount of sulfate radicals are present therein. If not, the pH was first adjusted to neutral with 2 M NaOH, and concentrated to about 10 mL on a rotary evaporator. The mixture was loaded on a P2 column, eluted with purified water, and the eluent was collected with a fraction collector. The absorbance at 232 nm was detected to determine the location of the heparin octasaccharide derivative, the collected solution was detected with $BaCl_2$ to determine whether a large amount of sulfate radicals are present therein, and the salt-free fraction of the heparin octasaccharide sulfated derivative was collected. The material was concentrated to about 10 mL, loaded on a Dowex cation exchange column, eluted with purified water, the eluent was collected with a fraction collector, and the absorbance at 232 nm was detected to determine the location of the heparin octasaccharide derivative. The fraction comprising the heparin octasaccharide derivative was collected, carefully adjusted to pH 7.0 with 0.1 M high-purity NaOH solution, and concentrated and lyophilized to obtain 20% sulfated heparin octasaccharide.

For 0.54 g of the starting material of heparin octasaccharide, heparin oligosaccharides with the indicated sulfation

degrees can be obtained in a controllable manner by adjusting the feed amount of $(CH_3)_3N{\bullet}SO_3$ in the starting materials of the reaction and adjusting the reaction temperature and the reaction time as shown in Table 1.

Table 1

| Sulfation Degree | Starting Material | Reaction Temperature | Reaction Time |
|---|---|---|---|
| 20% sulfation | 0.835g $(CH_3)_3N{\bullet}SO_3$ | 80 °C | 2-6h |
| 40% sulfation | 1.67g $(CH_3)_3N{\bullet}SO_3$ | 90°C | 2-6h |
| 60% sulfation | 3.34g $(CH_3)_3N{\bullet}SO_3$ | 90°C | 4-8h |
| 100% sulfation | 5.01g $(CH_3)_3N{\bullet}SO_3$ | 100°C | 6-10h |

For example, heparin oligosaccharides with corresponding sulfation degrees can be obtained through the adjustment of the reaction conditions and the feed amount as shown below.

The amount of $(CH_3)_3N{\bullet}SO_3$ was adjusted to 1.67 g, the reaction temperature was adjusted to 90°C, the reaction time was adjusted to 4 h, and the remaining procedures were the same, so as to obtain 40% sulfated heparin octasaccharide.

The amount of $(CH_3)_3N{\bullet}SO_3$ was adjusted to 3.34 g, the reaction temperature was adjusted to 90°C, the reaction time was adjusted to 6 h, and the remaining procedures were the same, so as to obtain 60% sulfated heparin octasaccharide.

The amount of $(CH_3)_3N{\bullet}SO_3$ was adjusted to 5.01 g, the reaction temperature was adjusted to 100°C, the reaction time was adjusted to 8 h, and the remaining procedures were the same, so as to obtain 80% sulfated heparin octasaccharide.

FIG. 5 shows HSQC spectra and the corresponding one-dimensional hydrogen spectra of the heparin octasaccharide (A) and 40% sulfated heparin octasaccharide (B) ($\delta_H$ ppm 6.1 to 3.165/$\delta_C$ ppm 112 to 52.6). It can be seen from the graph that after the sulfation reaction, the signal peaks of the heparin octasaccharide change significantly, and there are many new signals, indicating the presence of structures different from natural heparin, which is consistent with the expectation of sulfated derivatization.

Example 3. Heparanase inhibitory activity of 40% and 60% sulfated heparin octasaccharides

The heparanase inhibitory activity of sulfated heparin oligosaccharides was determined with the method as described in the reference [Hammond E, Li CP, Ferro V. Development of a colorimetric assay for heparanase activity suitable for kinetic analysis and inhibitor screening. Anal. Biochem. 2011; 396:112-116]. Specifically, the experimental procedure were as follows.

The reaction solution of the test group comprised 40 mM sodium acetate buffer (pH 5.0), 100 mM fondaparinux, and a specific concentration of the sulfated heparin octasaccharide, and in the reaction solution of the control group, the sulfated heparin octasaccharide was replaced with SST0001 control at a same concentration as that of the sulfated heparin octasaccharide. 100 μL of the reaction solution of the test group or the control group was added to each well in a 96-well plate, and heparanase was respectively added to a final concentration of 140 pM, to start the reaction. The 96-well plate was sealed with a tape, and incubated at 37°C for 2 to 24 h. After the reaction was complete, 100 μL of a solution containing 1.69 mM WST-1 in 0.1 M NaOH was added to stop the reaction. The 96-well plate was resealed and incubated at 60°C for 60 min. After cooling to room temperature, the absorbance value at 584 nm was measured. The inhibitory rate of the agent was calculated according to the following method:

$$\text{Inhibitory rate} = (1\text{-the absorbance value of a sample/the absorbance value of a control})\times 100\%$$

The heparanase inhibitory activity of the sulfated heparin oligosaccharides measured according to the above method is shown in FIG. 6. It can be seen from the graph that the half maximal inhibitory concentration (IC50) of 40% sulfated heparin octasaccharide (Hep8-40%) on heparanase was 43 ng/mL, and the half maximal inhibitory concentration (IC50) of 60% sulfated heparin octasaccharide (Hep8-60%) on heparanase was 57 ng/mL.

Example 4. Cell adhesion assay of 20% and 60% sulfated heparin octasaccharides

In this example, the following method was employed to determine the cell adhesion of 20% and 60% sulfated heparin octasaccharides.

(1) coating the basement membrane: 2 solutions were prepared with sterile double distilled water respectively: 10 g/L of BSA (1%), and 50 mg/L of Matrigel, 1:8 dilution; Matrigel was added to a 96-well culture plate at 50 μL/well respectively, and incubated at 4°C overnight;

(2) hydrating the basement membrane: the residual liquid was aspirated from the culture plate, 50 μL of serum-free medium containing 10 g/L of BSA was add to each well, and allowed to stand at 37°C for 30 min.

(3) preparing cells: normally cultured HeLa cells were taken and washed with PBS for three times, and the cells were divided into a control group (no drug but only a corresponding volume of PBS was added) and test groups (heparin and sulfated heparin octasaccharide were added, both at a concentration of 62.5 μg/mL), and cultured for 24 h.

(4) inoculating cells: tumor cells normally cultured or treated with heparin/HS oligosaccharide derivatives in step (3) for 24 h were taken, and washed with PBS for three times before digested with 0.5 mL of pancreatin. The digestion was closely monitored, and after the digestion, 5 mL of the culture medium was added, and the cells were dispersed by thoroughly pipetting to form a single cell suspension. The cells were counted, and the concentration of the cell suspension was adjusted according to the counting result to achieve a cell density of $10^5$ cells/mL. The cell suspension was inoculated at 100 μL per well to a 96-well culture plate coated with Matrigel, and samples were processed in triplicate;

(5) culturing cells: the cells were cultured in a carbon dioxide incubator at 37°C for 1 h, and washed with PBS once after the culture liquid in each well was discarded. 200 μL per well of fresh culture medium was then added, and the plated was observed and photographed.

(6) detecting: 10 μL of a CCK-8 dye solution was added to each well, and the plate was incubated in a carbon dioxide incubator at 37°C for 3 hours, and read with a multifunctional microplate reader. The inhibitory rate was calculated according to the following formula, with the heparin control group as a reference.

$$\text{Inhibitory rate}=(1-\text{the absorbance value of the treatment group/the absorbance value of the control group})\times 100\%$$

According to the above method, the inhibitory rate of heparin on HeLa cell adhesion was 8.3%, the inhibitory rate of 20% sulfated heparin octasaccharide (Hep8-20%) on HeLa cell adhesion was 45.3%, and the inhibitory rate of 60% sulfated heparin octasaccharide (Hep8-60%) on HeLa cell adhesion was 37.9%.

Example 5. Cellular migration assay with 40% and 60% sulfated heparin octasaccharides

In this example, cell migration with 40% and 60% sulfated heparin octasaccharides was determined by the following method.

(1) before the test, a Transwell chamber was placed in a 24-well plate, 600 μL of a DMEM liquid medium was added to the lower chamber, 100 μL of a DMEM liquid medium was added to the upper chamber, and the chamber was left in an incubator overnight for further use;

(2) preparation of cells: HeLa cells in logarithmic growth phase were taken and digested with pancreatin, the cell concentration was counted, and the cells were divided into a control group (no heparin or heparin oligosaccharides but only a corresponding volume of PBS was added) and test groups (heparin and sulfated heparin octasaccharide were added, both at a concentration of 62.5 μg/mL). The cells were diluted with a serum-free DMEM medium containing 2.5% BSA and serum-free, 2.5% BSA DMEM medium containing a corresponding heparin oligosaccharide to adjust the cell concentration to $2.5\times10^5$ cells/mL.

(3) 800 μL of a culture medium containing 5% FBS was added to the lower chamber of the Transwell chamber.

(4) 400 μL of the cell suspension in step (2) was added to the upper chamber of the Transwell chamber.

(5) The chamber was incubated in an incubator at 37°C under 5% $CO_2$ for 8 hours.

(6) The liquid in the upper chamber was carefully removed, and the inner surface of the chamber membrane was gently wiped with a cotton swab to remove non-migrating cells. It should be noted that the operation should be gentle so as to avoid damaging the chamber membrane.

(7) The chamber was transferred to another 24-well plate containing 400 μL of cell dye liquor, and the cells were stained at room temperature for 10 minutes.

(8) The Transwell chamber was gently washed with distilled water for 3 to 5 times, and left at room temperature for natural-air drying.

(9) The cells were counted under a microscope and photographed.

(10) The chamber was transferred to another clean 24-well plate, 200 μL of a cell lysis solution was added to each well, and the chamber was placed on a shaker.

(11) After incubated at room temperature for 10 minutes, 100 μL of the cell lysis solution was added to a 96-well plate, and the absorbance value was read at 560 nm. With the heparin control group as a reference, the transfer rate and inhibitory rate were calculated according to the following formulae:

Transfer rate=the absorbance value of the treatment group/the absorbance value of the control group×100%

Inhibitory rate=[1- (the adhesion rate of the treatment group/the adhesion rate of the control group)]×100%

According to the above method, the results of cell adhesion are shown in FIG. 7, showing that that the inhibitory rate of heparin on HeLa cell migration is 12.6%, the inhibitory rate of 40% sulfated heparin octasaccharide (Hep8-40%) on HeLa cell migration is 57.3%, and the inhibitory rate of 60% sulfated heparin octasaccharide (Hep8-60%) on HeLa cell migration is 43.5%.

Example 6. Anti-tumor metastasis activity of 40% and 60% sulfated heparin octasaccharides

In this example, the anti-tumor metastasis activity of 40% and 60% sulfated heparin octasaccharides was determined according to the following method.

B16-BL6 mouse melanoma cells (2×105) were injected into C57BL/6 mice via the tail vein, and the mice were divided into a control group (no heparin oligosaccharide was added, and PBS was employed as control) and test groups (40% sulfated heparin octasaccharide was added, 200 μg for each mouse), 10 mice per group. After 3 weeks, the mice were dissected, the lungs of the mice were fixed in the Bouin's solution, and the numbers of tumors in the lungs of the mice were calculated. We detected the formation of metastatic tumor weekly through an IVIS-200 fluorescence imaging system by employing luciferase-labeled B16-BL6 mouse melanoma cells. Pictures were taken 10 min after intraperitoneal injection of 2.5 mg of fluorescein.

According to the above method, the results of anti-tumor metastasis activity are shown in FIG. 8. It can be seen from the graph that the inhibitory rate of heparin on tumor metastasis in mice is 17.3%, the inhibitory rate of 40% sulfated heparin octasaccharide (Hep8-40%) on tumor metastasis in mice is 65.1%, and the inhibitory rate of 60% sulfated heparin octasaccharide (Hep8-60%) on tumor metastasis in mice is 56.2%.

The applicant hereby states that the above-mentioned examples in the present invention are provided by way of illustration of the sulfated heparin oligosaccharide of the present invention, the preparation method and use thereof, but the present invention is not limited to the above-mentioned examples, i.e. it is not intended that the present invention

has to rely on the above-mentioned examples for implementation. It should be appreciated by a person skilled in the art that any modification to the present invention, equivalent replacement of materials employed and addition of an adjunct ingredient in the present invention, as well as selection of specific embodiments *etc.* would fall within the protection and disclosure scope of the present invention.

## Claims

1. A sulfated heparin oligosaccharide, **characterized in that** the sulfated heparin oligosaccharide molecule contains an unsaturated double bond resulting from enzymolysis by heparinase at the non-reducing end thereof, comprises an uronic acid derivative and a glycosylamine derivative, and has a structure represented by Formula I:

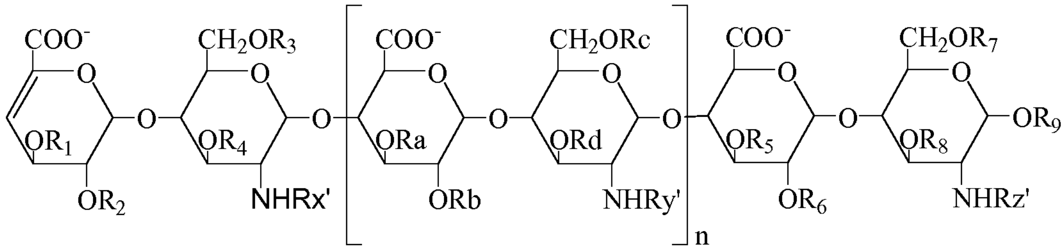

Formula I

wherein $R_1$, $R_2$, $R_3$, $R_4$, $R_5$, $R_6$, $R_7$, $R_8$, $R_9$, $R_a$, $R_b$, $R_c$ and $R_d$ are independently $SO_3^-$ or H; $R_x$', $R_y$' and $R_z$' are independently $COCH_3$ or $SO_3^-$, and n is 1 to 3.

2. The sulfated heparin oligosaccharide according to claim 1, **characterized in that** in Formula I, the number of sulfonic groups in each disaccharide unit is no less than 2 on average, the number of acetyl groups in each disaccharide unit is no more than 0.5 on average, and the numbers of sulfonic groups at position 6 and position 3 in glucosamine in each disaccharide unit are both no less than 0.5 on average;
preferably, the uronic acid in Formula I is glucuronic acid or iduronic acid; and
preferably, the cation forming a salt with the carboxyl and/or sulfonic group contained in Formula I is selected from the group consisting of $Na^+$, $K^+$ or $Ca^{2+}$.

3. The sulfated heparin oligosaccharide according to claim 1 or 2, **characterized in that** the sulfated heparin oligosaccharide is any one of, or a combination of at least two of, the compounds having the following structures:

DP6, a heparin hexasaccharide derivative:

$\Delta U_{3S}\text{-}A_{NS3S}\text{-}I\text{-}A_{NS3S}\text{-}G_{3S}\text{-}A_{NS3S6S}$
$\Delta U_{3S}\text{-}A_{NS}\text{-}I_{3S}\text{-}A_{NS3S}\text{-}G_{3S}\text{-}A_{NS3S6S}$
$\Delta U_{2S3S}\text{-}A_{NS3S6S}\text{-}I_{3S}\text{-}A_{NS3S}\text{-}G_{2S3S}\text{-}A_{NS3S6S}$
$\Delta U_{2S3S}\text{-}A_{NS3S6S}\text{-}I_{3S}\text{-}A_{NS3S6S}\text{-}G_{3S}\text{-}A_{NS3S6S}$
$\Delta U_{2S3S}\text{-}A_{NS3S6S}\text{-}I_{3S}\text{-}A_{NS3S6S}\text{-}G_{2S3S}\text{-}A_{NS3S6S}$
$\Delta U_{2S3S}\text{-}A_{NS3S6S}\text{-}I_{2S3S}\text{-}A_{NS3S6S}\text{-}G_{2S3S}\text{-}A_{NS3S6S}$

DP8, a heparin octasaccharide derivative:

$\Delta U_{3S}\text{-}A_{NS3S}\text{-}I\text{-}A_{NS3S}\text{-}I_{3s}\text{-}A_{NS6S}\text{-}G_{3S}\text{-}A_{NS3S6S}$
$\Delta U_{3S}\text{-}A_{NS}\text{-}I_{3S}\text{-}A_{NS3S}\text{-}I_{3S}\text{-}A_{NS6S}\text{-}G_{3S}\text{-}A_{NS3S6S}$
$\Delta U_{2S3S}\text{-}A_{NS3S6S}\text{-}I_{3S}\text{-}A_{NS3S}\text{-}I_{3S}\text{-}A_{NS6S}\text{-}G_{2S3S}\text{-}A_{NS3S6S}$
$\Delta U_{2S3S}\text{-}A_{NS3S6S}\text{-}I_{3S}\text{-}A_{NS3S6S}\text{-}I_{3S}\text{-}A_{NS6S}\text{-}G_{3S}\text{-}A_{NS3S6S}$
$\Delta U_{2S3S}\text{-}A_{NS3S6S}\text{-}I_{3S}\text{-}A_{NS3S6S}\text{-}I_{3S}\text{-}A_{NS6S}\text{-}G_{2S3S}\text{-}A_{NS3S6S}$
$\Delta U_{2S3S}\text{-}A_{NS3S6S}\text{-}I_{2S3S}\text{-}A_{NS3S6S}\text{-}I_{2S3S}\text{-}A_{NS6S}\text{-}G_{2S3S}\text{-}A_{NS3S6S}$

DP10, a heparin decasaccharide derivative:

$\Delta U_{3S}$-$A_{NS3S}$-I-$A_{NS3S}$-I-$A_{NS6S}$-I-$A_{NS6S}$-$G_{3S}$-$A_{NS3S6S}$
$\Delta U_{3S}$-$A_{NS}$-$I_{3S}$-$A_{NS3S}$-$I_{3S}$-$A_{NS6S}$-$I_{3S}$-$A_{NS6S}$-$G_{3S}$-$A_{NS3S6S}$
$\Delta U_{2S3S}$-$A_{NS3S6S}$-$I_{3S}$-$A_{NS3S}$-$I_{3S}$-$A_{NS6S}$-$I_{3S}$-$A_{NS6S}$-$G_{3S}$-$A_{NS3S6S}$
$\Delta U_{2S3S}$-$A_{NS3S6S}$-$I_{3S}$-$A_{NS3S6S}$-$I_{3S}$-$A_{NS6S}$-$I_{3S}$-$A_{NS6S}$-$G_{2S3S}$-$A_{NS3S6S}$
$\Delta U_{2S3S}$-$A_{NS3S6S}$-$I_{2S3S}$-$A_{NS3S6S}$-$I_{2S3S}$-$A_{NS6S}$-$I_{2S3S}$-$A_{NS6S}$-$G_{2S3S}$-$A_{NS3S6S}$
$\Delta U_{2S3S}$-$A_{NS3S6S}$-$I_{2S3S}$-$A_{NS3S6S}$-$I_{2S3S}$-$A_{NS3S6S}$-$I_{2S3S}$-$A_{NS3S6S}$-$G_{2S3S}$-$A_{NS3S6S}$.

**4.** A preparation method for the sulfated heparin oligosaccharide according to any one of claims 1 to 3, **characterized in that** the method comprises the following steps:

(1) degrading heparin with heparinase, separating and purifying to obtain a heparin oligosaccharide; and
(2) sulfating the heparin oligosaccharide obtained in step (1) with a sulfating reagent to obtain the sulfated heparin oligosaccharide.

**5.** The preparation method according to claim 4, **characterized in that** the heparinase in step (1) is heparinase I.

**6.** The preparation method according to claim 4 or 5, **characterized in that** a buffer, preferably a Tris-HCl buffer, pH 7.0, needs to be added when heparin is degraded with heparinase in step (1);
preferably, the amount of heparinase added in step (1) is 15 to 25 IU/g heparin, preferably 18 to 23 IU/g heparin;
preferably, the temperature for the degradation of heparin with heparinase in step (1) is 4°C to 37°C, preferably 8°C to 25°C;
preferably, the time for the degradation in step (1) is 8 to 24 hours, preferably 10 to 20 hours;
preferably, the degradation of heparin with heparinase in step (1) comprises inactivation at 95°C for 5 to 10 min, preferably 5 to 8 min, and more preferably 6 min, after the degradation;
preferably, the separation in step (1) comprises ultrafiltration, preferably ultrafiltration with a 10 KDa ultrafiltration centrifuge tube; and
preferably, the purification in step (1) is separation and purification through column chromatography.

**7.** The preparation method according to any one of claims 4 to 6, **characterized in that** before the sulfation in step (2), the heparin oligosaccharide obtained in step (1) is subjected to swelling treatment; and
preferably, the solvent used for the swelling treatment is DMF.

**8.** The preparation method according to any one of claims 4 to 7, **characterized in that** the sulfating reagent in step (2) is $(CH_3)_3N \cdot SO_3$;
preferably, relative to 1 g of the heparin oligosaccharide, the amount of the sulfating reagent is 1 to 10 g;
preferably, the temperature for the sulfation in step (2) is 60°C to 120°C; and
preferably, the time for the sulfation in step (2) is 1 to 12 hours.

**9.** The preparation method according to any one of claims 4 to 8, **characterized in that** the method comprises the following steps:

(1) degrading heparin with heparinase I, which is added at an amount of 15 to 25 IU/g heparin, at 4°C to 37°C for 8 h to 24 h in a buffer being a Tris-HCl buffer, pH 7.0, and after the degradation, inactivating at 95°C for 5 to 10 min, ultrafiltering with a 10 KDa ultrafiltration centrifuge tube, and then separating and purifying through column chromatography to obtain the heparin oligosaccharide; and
(2) sulfating the heparin oligosaccharide obtained in step (1) at 60°C to 120°C for 1 to 12 hours with a sulfating reagent to obtain the sulfated heparin oligosaccharide.

**10.** Use of the sulfated heparin oligosaccharide according to any one of claims 1-3 in the manufacture of a medicament for anti-tumor metastasis.

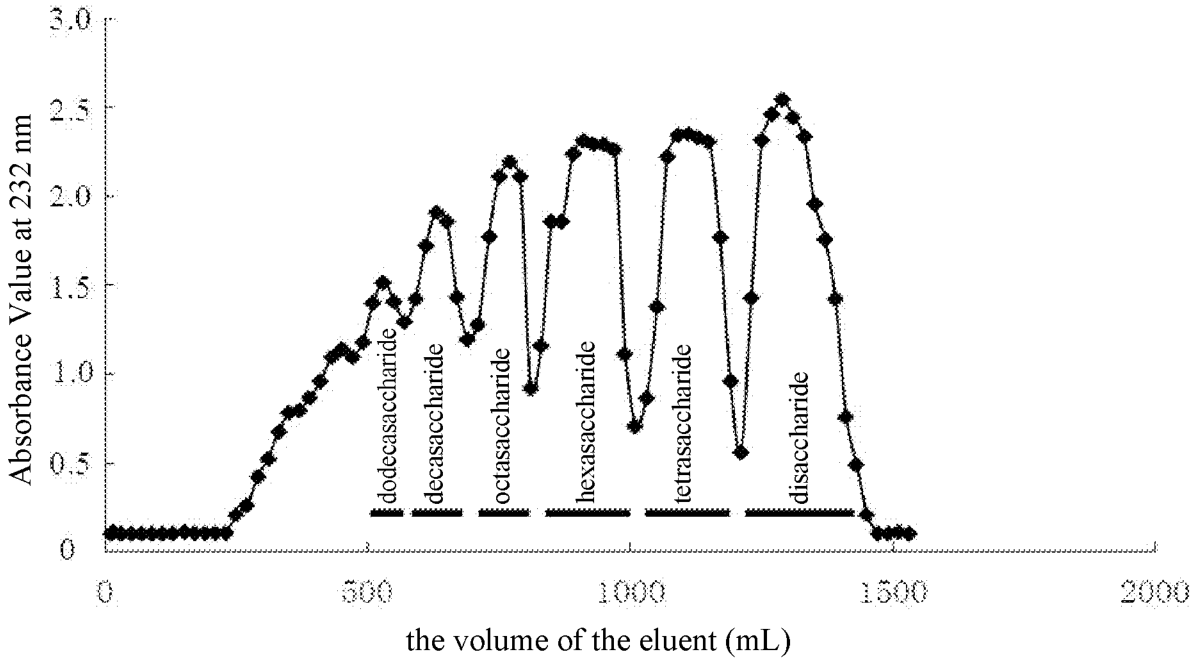
3.0
2.5
2.0
1.5
1.0
0.5
0
Absorbance Value at 232 nm
dodecasaccharide
decasaccharide
octasaccharide
hexasaccharide
tetrasaccharide
disaccharide
0
500
1000
1500
2000
the volume of the eluent (mL)

FIG.1

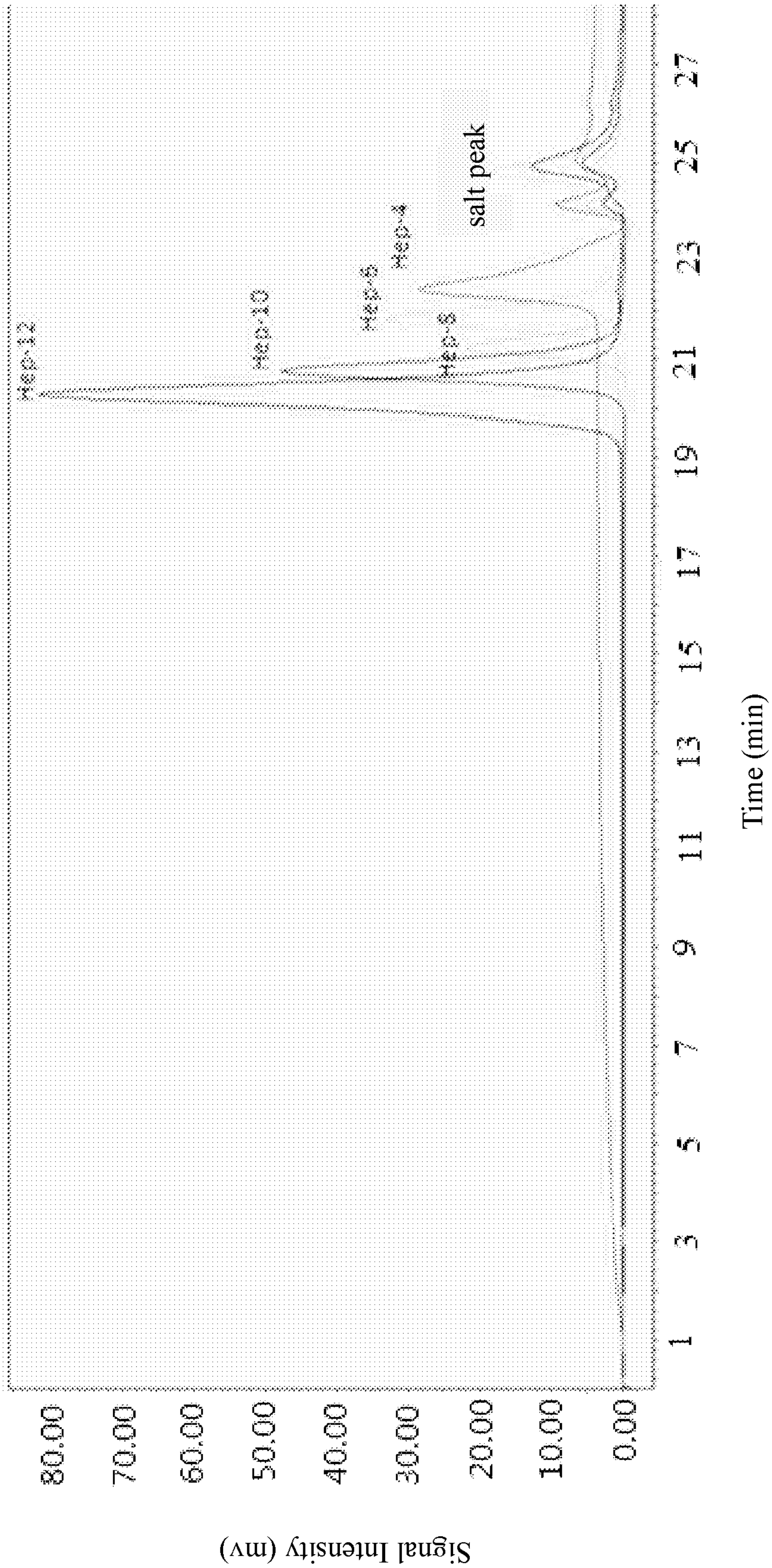
Signal Intensity (mv)
80.00
70.00
60.00
50.00
40.00
30.00
20.00
10.00
0.00
Hep-12
Hep-10
Hep-8
Hep-6
Hep-4
salt peak
1
3
5
7
9
11
13
15
17
19
21
23
25
27
Time (min)

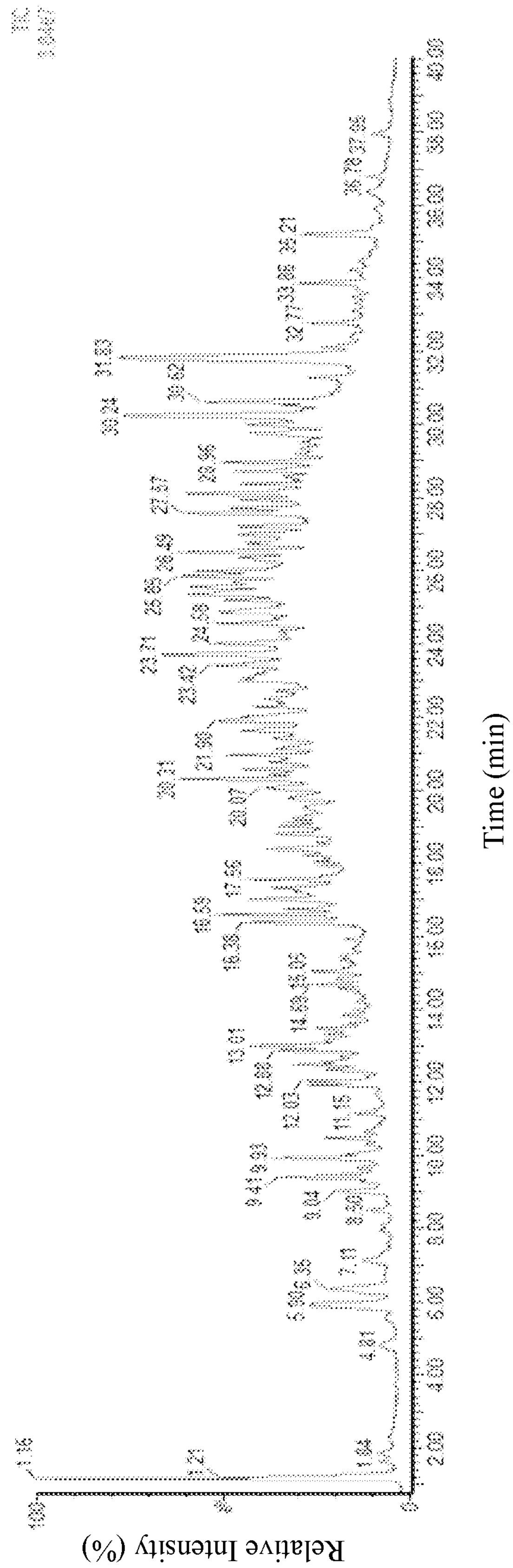


FIG.3

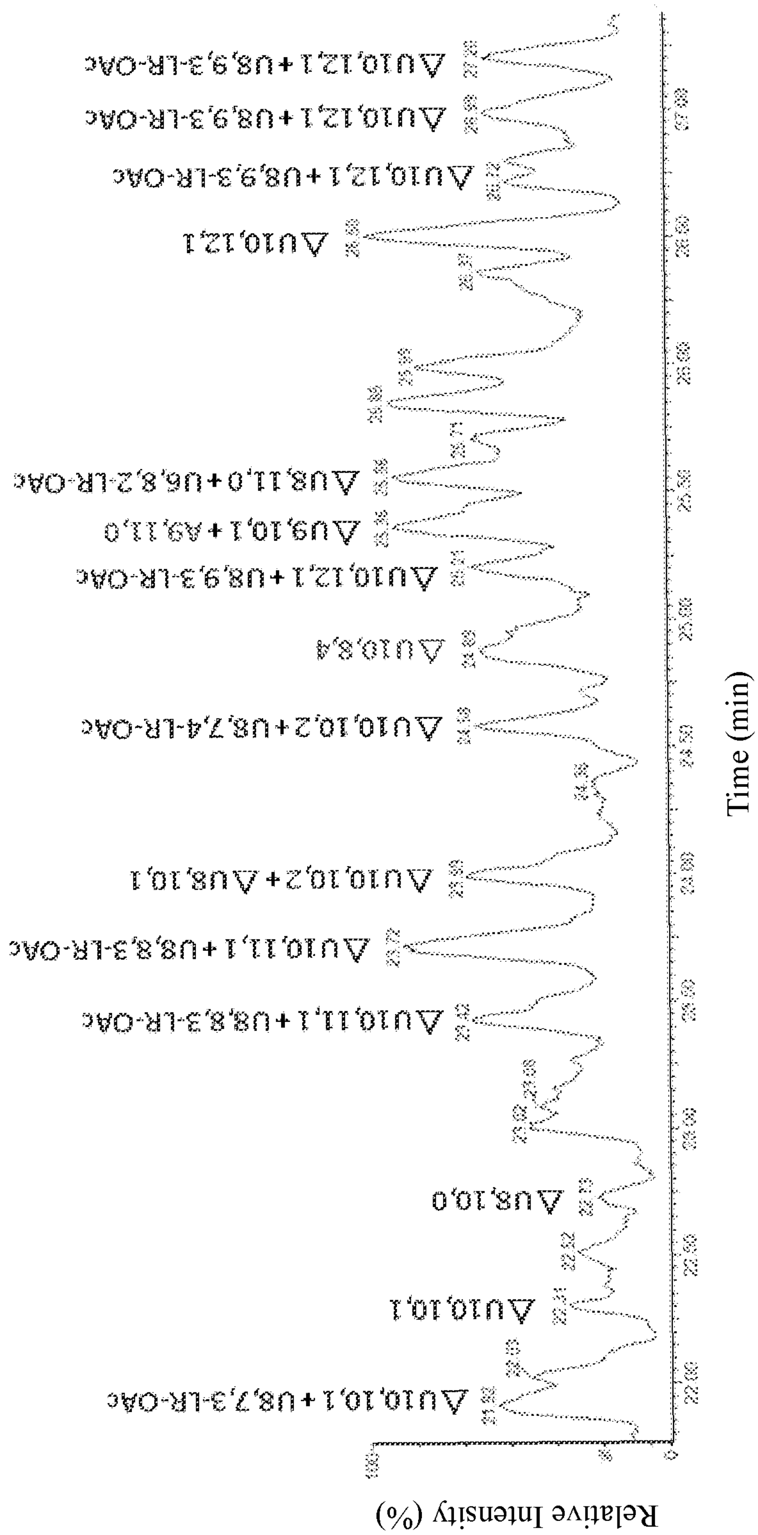
Relative Intensity (%)
Time (min)
ΔU10,10,1+U8,7,3-LR-OAc
ΔU10,10,1
ΔU8,10,0
ΔU10,11,1+U8,8,3-LR-OAc
ΔU10,11,1+U8,8,3-LR-OAc
ΔU10,10,2+ΔU8,10,1
ΔU10,10,2+U8,7,4-LR-OAc
ΔU10,8,4
ΔU10,12,1+U8,9,3-LR-OAc
ΔU9,10,1+A9,11,0
ΔU8,11,0+U6,8,2-LR-OAc
ΔU10,12,1
ΔU10,12,1+U8,9,3-LR-OAc
ΔU10,12,1+U8,9,3-LR-OAc
ΔU10,12,1+U8,9,3-LR-OAc

FIG.4

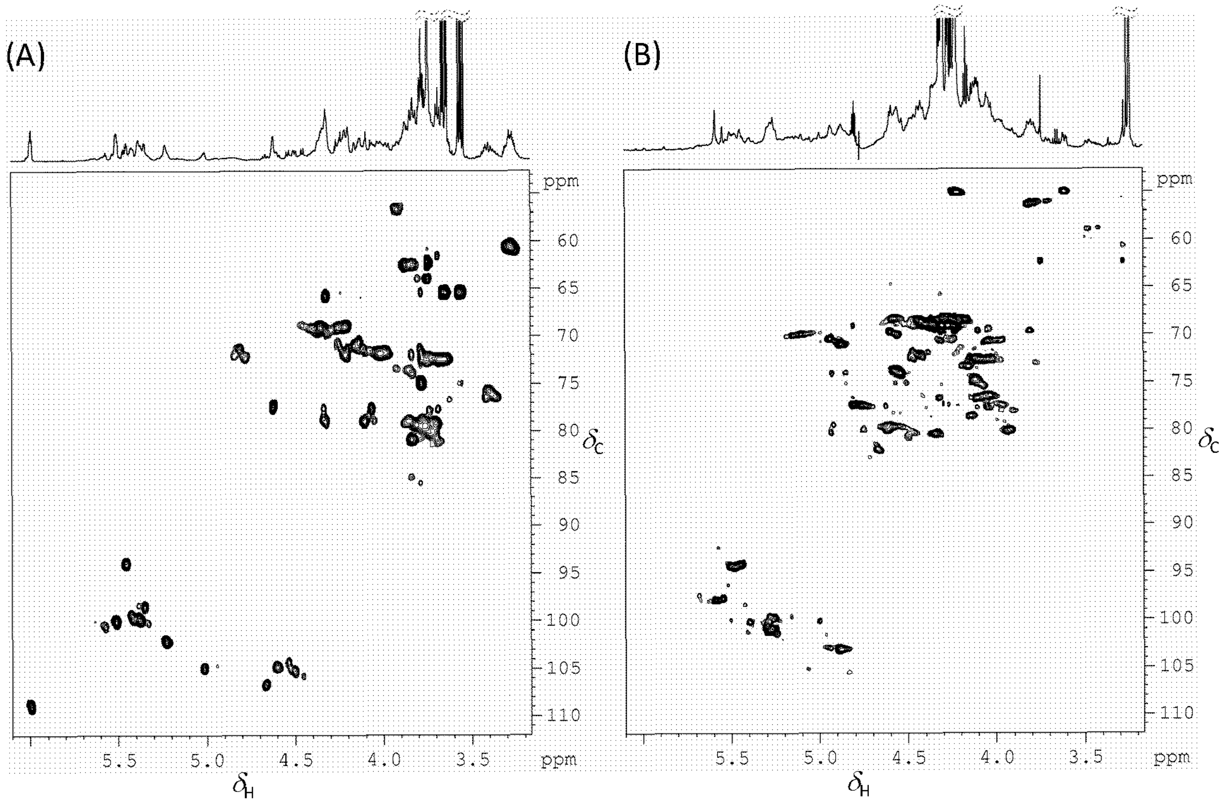
(A)
(B)
ppm
60
65
70
75
80
85
90
95
100
105
110
δC
5.5
5.0
4.5
4.0
3.5
ppm
δH

FIG.5

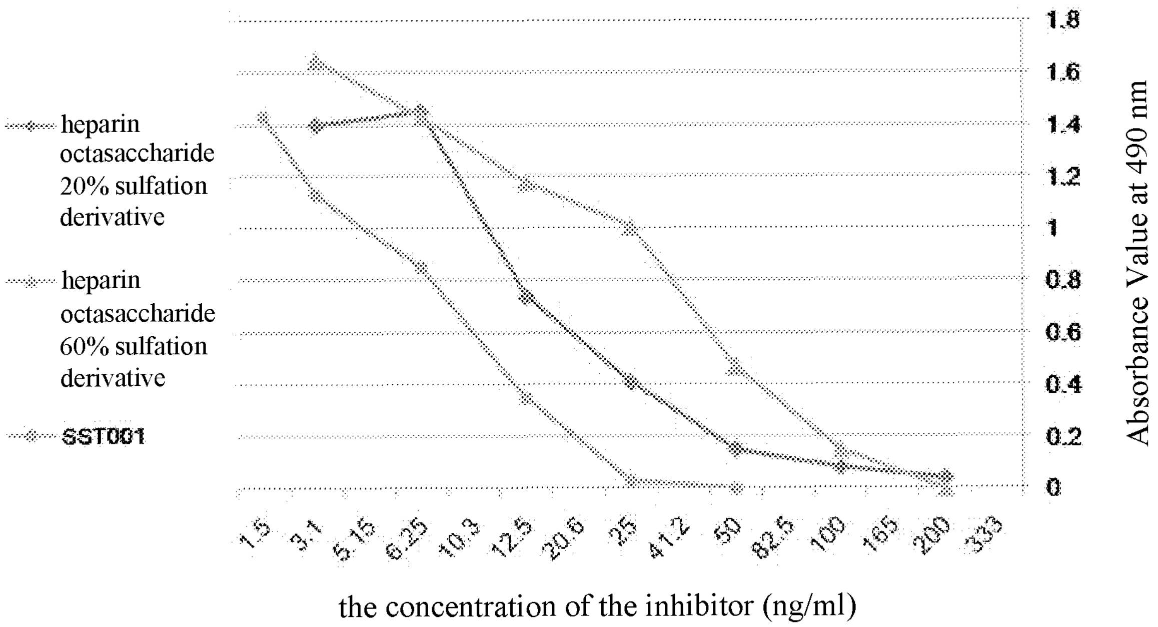
heparin octasaccharide 20% sulfation derivative
heparin octasaccharide 60% sulfation derivative
SST001
1.8
1.6
1.4
1.2
1
0.8
0.6
0.4
0.2
0
Absorbance Value at 490 nm
1.5
3.1
5.15
6.25
10.3
12.5
20.6
25
41.2
50
82.5
100
165
200
333
the concentration of the inhibitor (ng/ml)

FIG.6

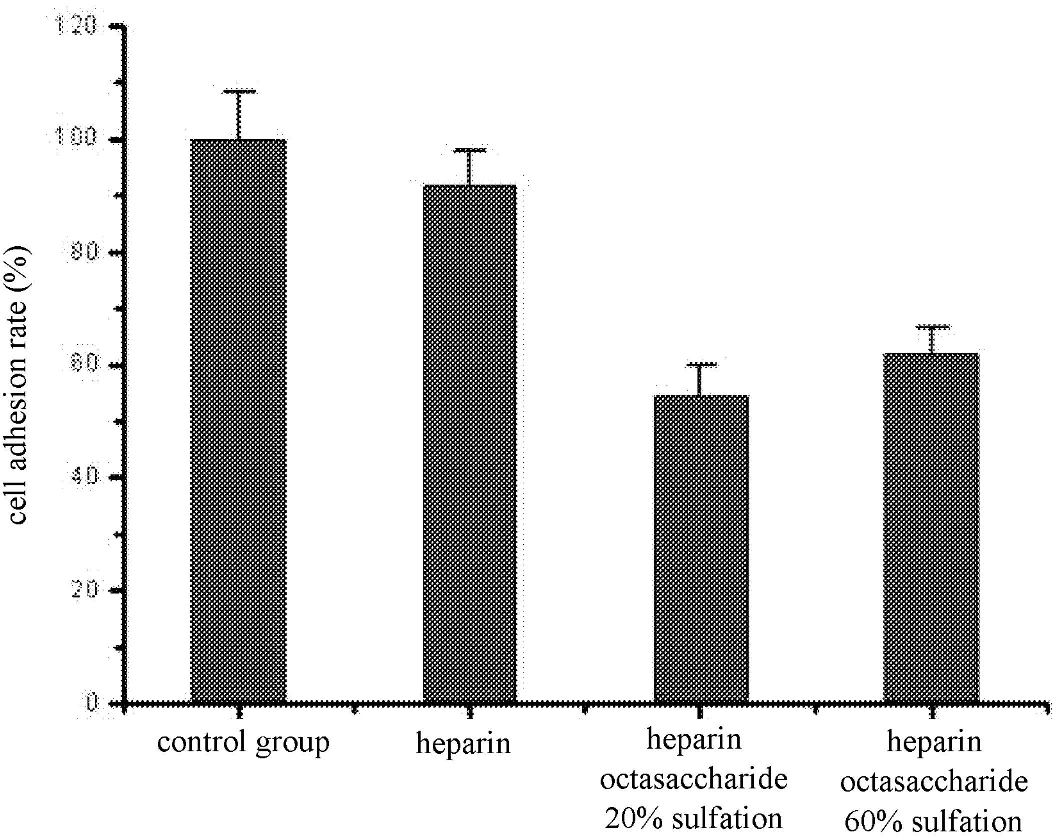
120
100
80
60
40
20
0
cell adhesion rate (%)
control group
heparin
heparin octasaccharide 20% sulfation
heparin octasaccharide 60% sulfation

FIG.7

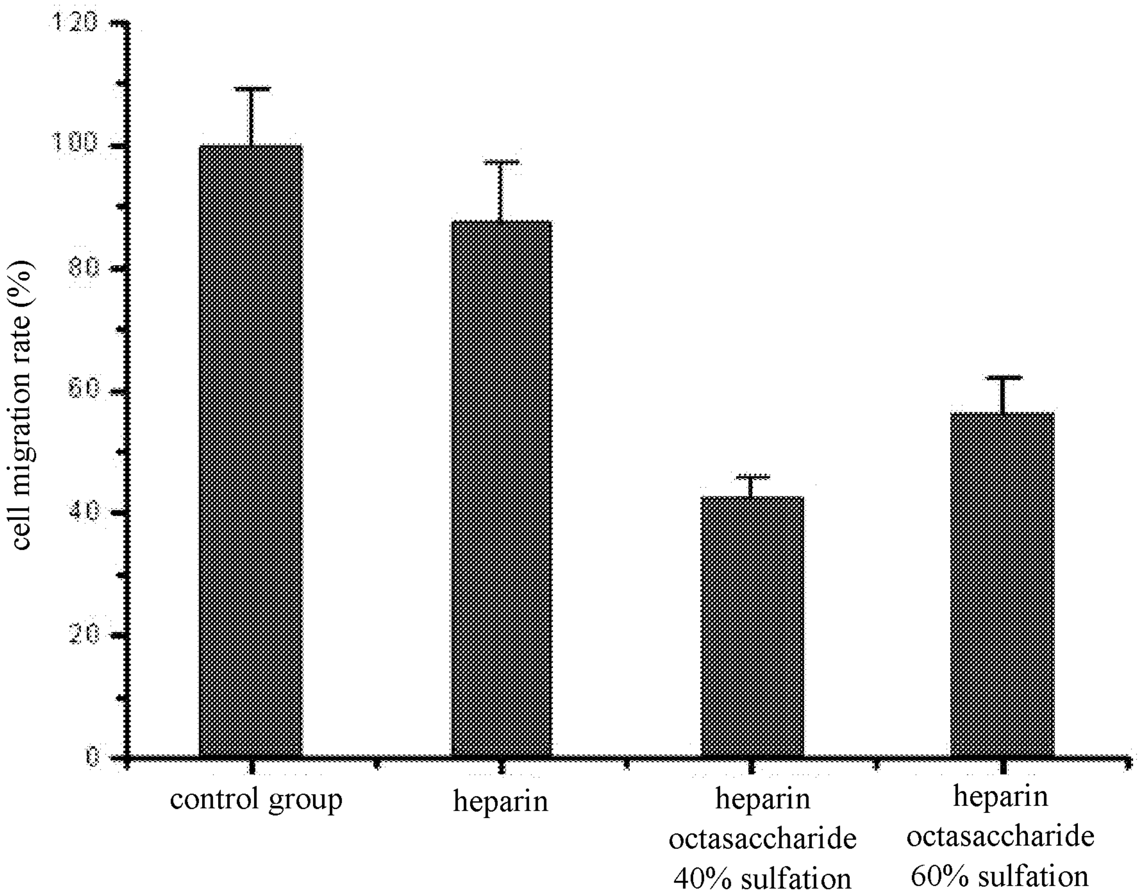
cell migration rate (%)
120
100
80
60
40
20
0
control group
heparin
heparin octasaccharide 40% sulfation
heparin octasaccharide 60% sulfation

FIG.8

# INTERNATIONAL SEARCH REPORT

International application No.
**PCT/CN2015/099901**

**A. CLASSIFICATION OF SUBJECT MATTER**

C08B 37/10 (2006.01) i; A61K 31/727 (2006.01) i; A61P 35/00 (2006.01) i

According to International Patent Classification (IPC) or to both national classification and IPC

**B. FIELDS SEARCHED**

Minimum documentation searched (classification system followed by classification symbols)

C08B; C12P; A61K; A61P

Documentation searched other than minimum documentation to the extent that such documents are included in the fields searched

Electronic data base consulted during the international search (name of data base and, where practicable, search terms used)

CNPAT, CMQ, WPI, EPODOC, ISI, CA: HEPALINK PHARMACEUTICAL; sulfuric acid, sulfat+, heparin, oligosaccharide, enzyme, degradat+, LMWH, low molecule, SO3, sulfur trioxide trimethylamine

**C. DOCUMENTS CONSIDERED TO BE RELEVANT**

| Category* | Citation of document, with indication, where appropriate, of the relevant passages | Relevant to claim No. |
|---|---|---|
| X | CN 101495517 A (MOMENTA PHARMACEUTICALS, INC.), 29 July 2009 (29.07.2009), description, page 6, paragraph 3 and page 20, paragraph 3 to page 21, paragraph 1 | 1-3, 10 |
| Y | CN 101495517 A (MOMENTA PHARMACEUTICALS, INC.), 29 July 2009 (29.07.2009), description, page 6, paragraph 3 and page 20, paragraph 3 to page 21, paragraph 1 | 4-9 |
| Y | CN 103288981 A (SHENZHEN HEPALINK PHARMACEUTICAL CO., LTD.), 11 September 2013 (11.09.2013), description, paragraphs [0004] and [0007]-[0009] | 4-9 |
| X | CN 1798771 A (AVENTIS PHARMA S.A.), 05 July 2006 (05.07.2006), claim 1 | 1-2 |
| X | CN 103788232 A (SHENZHEN HEPALINK PHARMACEUTICAL CO., LTD.), 14 May 2014 (14.05.2014), claims 1 and 2 | 1 |
| E | CN 105504097 A (SHENZHEN HEPALINK PHARMACEUTICAL CO., LTD.), 20 April 2016 (20.04.2016), claims 1-10 | 1-10 |
| A | US 6190875 B1 (INSIGHT STRATEGY & MARKETING et al.), 20 February 2001 (20.02.2001), description, column 17, line 30 to column 18, line 27 | 1-10 |

☐ Further documents are listed in the continuation of Box C.    ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 07 September 2016 (07.09.2016) | **22 September 2016 (22.09.2016)** |

Name and mailing address of the ISA/CN:
State Intellectual Property Office of the P. R. China
No. 6, Xitucheng Road, Jimenqiao
Haidian District, Beijing 100088, China
Facsimile No.: (86-10) 62019451

Authorized officer
**HAN, Xiaojie**
Telephone No.: (86-10) **010-82246864**

**INTERNATIONAL SEARCH REPORT**
Information on patent family members

International application No.
**PCT/CN2015/099901**

| Patent Documents referred in the Report | Publication Date | Patent Family | Publication Date |
|---|---|---|---|
| CN 101495517 A | 29 July 2009 | EP 2447285 A2 | 02 May 2012 |
| | | US 8609632 B2 | 17 December 2013 |
| | | WO 2007140231 A2 | 06 December 2007 |
| | | JP 2009538386 A | 05 November 2009 |
| | | WO 2007140231 A3 | 04 December 2008 |
| | | AU 2007267561 B2 | 17 May 2012 |
| | | CA 2652205 A1 | 06 December 2007 |
| | | AU 2007267561 A1 | 06 December 2007 |
| | | EP 2019843 A2 | 04 February 2009 |
| | | EP 2404939 A3 | 21 March 2012 |
| | | CN 101495517 B | 10 October 2012 |
| | | US 2007287683 A1 | 13 December 2007 |
| | | EP 2404939 A2 | 11 January 2012 |
| | | US 2010305022 A1 | 02 December 2010 |
| | | EP 2447285 A3 | 16 January 2013 |
| | | RU 2008151420 A | 27 June 2010 |
| | | RU 2451515 C2 | 27 May 2012 |
| | | IN 200804734 P2 | 13 March 2009 |
| | | BR PI0713123 A2 | 10 April 2012 |
| CN 103288981 A | 11 September 2013 | None | |
| CN 1798771 A | 05 July 2006 | ZA 200502787 B | 18 October 2005 |
| | | JP 5389140 B2 | 15 January 2014 |
| | | ZA 200502787 A | 18 October 2005 |
| | | NO 20052170 A | 07 July 2005 |
| | | EC SP055722 A | 06 July 2005 |
| | | EP 1556414 A1 | 27 July 2005 |
| | | OA 12940 A | 13 October 2006 |
| | | NZ 539229 A | 28 March 2008 |
| | | SV 2004001626 A | 08 March 2004 |
| | | CN 101810637 A | 25 August 2010 |
| | | WO 2004033503 A1 | 22 April 2004 |
| | | IL 167868 A | 31 May 2010 |
| | | JP 2012051926 A | 15 March 2012 |
| | | JP 5021898 B2 | 12 September 2012 |
| | | PT 1556414 E | 21 May 2012 |
| | | GT 200300181 A | 24 March 2004 |
| | | CA 2501546 A1 | 22 April 2004 |
| | | FR 2845686 A1 | 16 April 2004 |
| | | PE 05072004 A1 | 13 October 2004 |
| | | CN 101812189 A | 25 August 2010 |
| | | AT 548393 T | 15 March 2012 |
| | | PA 8584201 A1 | 31 August 2004 |
| | | CN 101812190 A | 25 August 2010 |
| | | EP 1556414 B1 | 07 March 2012 |
| | | UA 81925 C2 | 25 February 2008 |
| | | CA 2501546 C | 03 April 2012 |
| | | AU 2003300161 B2 | 27 August 2009 |
| | | HN 2003000312 A | 20 October 2005 |
| | | TW 200418882 A | 01 October 2004 |
| | | TW I332010 B | 21 October 2010 |

Form PCT/ISA/210 (patent family annex) (July 2009)

**INTERNATIONAL SEARCH REPORT**
Information on patent family members

International application No.
**PCT/CN2015/099901**

| Patent Documents referred in the Report | Publication Date | Patent Family | Publication Date |
|---|---|---|---|
| | | MA 27398 A1 | 01 June 2005 |
| | | FR 2845686 B1 | 30 August 2013 |
| | | JP 2006517185 A | 20 July 2006 |
| | | ES 2383597 T3 | 22 June 2012 |
| | | NO 20052170 D0 | 03 May 2005 |
| | | CN 101812139 A | 25 August 2010 |
| | | MX PA05003321 A | 05 July 2005 |
| | | SI 1556414 T1 | 29 June 2012 |
| | | CN 101817940 A | 01 September 2010 |
| | | CY 1112808 T1 | 10 February 2016 |
| | | RS 20050274 A | 04 June 2007 |
| | | MY 142376 A | 30 November 2010 |
| | | DK 1556414 T3 | 25 June 2012 |
| | | RU 2332424 C2 | 27 August 2008 |
| | | AU 2003300161 A1 | 04 May 2004 |
| | | RU 2005113987 A | 27 January 2006 |
| | | BR 0315149 A | 16 August 2005 |
| | | UY 28016 A1 | 30 April 2004 |
| | | SG 110926 B | 30 September 2008 |
| | | US 8071570 B2 | 06 December 2011 |
| | | US 2011212915 A1 | 01 September 2011 |
| | | KR 1104107 B | 12 January 2012 |
| | | VN 10010772 B | 26 November 2012 |
| | | IN 200500569 P4 | 25 May 2007 |
| | | IN 219266 B | 06 June 2008 |
| | | MX 258770 B | 15 July 2008 |
| | | US 2011201573 A1 | 18 August 2011 |
| | | KR 20050061527 A | 22 June 2005 |
| | | US 2008182820 A1 | 31 July 2008 |
| | | US 2004171819 A1 | 02 September 2004 |
| | | US 8003623 B2 | 23 August 2011 |
| | | HK 1145031 A0 | 25 March 2011 |
| | | HK 1145015 A0 | 25 March 2011 |
| | | HK 1145029 A0 | 25 March 2011 |
| | | HK 1145030 A0 | 25 March 2011 |
| CN 103788232 A | 14 May 2014 | None | |
| CN 105504097 A | 20 April 2016 | None | |
| US 6190875 B1 | 20 February 2001 | CA 2335382 A1 | 20 January 2000 |
| | | JP 2002520029 A | 09 July 2002 |
| | | AU 4869799 A | 01 February 2000 |
| | | AU 758485 B2 | 20 March 2003 |
| | | WO 0003036 A1 | 20 January 2000 |
| | | NO 20010136 D0 | 09 January 2001 |
| | | NO 20010136 A | 09 March 2001 |
| | | EP 1097241 A1 | 09 May 2001 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101824100 A
- CN 104764847 A


### Non-patent literature cited in the description


- **HAMMOND E ; LI CP ; FERRO V.** Development of a colorimetric assay for heparanase activity suitable for kinetic analysis and inhibitor screening. *Anal. Biochem.,* 2011, vol. 396, 112-116